# EUROPEAN PATENT APPLICATION

(11) **EP 2 930 636 A1**
(43) Date of publication of application: **14.10.2015**
(21) Application number: 15162661.1
(22) Date of filing: 07.04.2015
(51) Int. Cl.: G06F 19/00

(54) **Method and system for the management of post-acquisition processing procedures related to biomedical images**

(30) Priority: 08.04.2014 IT GE20140035
(71) Applicant: Camelot Biomedical System srl, 16152 Genova (IT)
(72) Inventor: Basso, Curzio, 16146 Genova (IT); Santoro, Matteo, 16148 Genova (IT); Esposito, Mario, 16149 Genova (IT); Mosci, Sofia, 16124 Genova (IT)
(74) Representative: Karaghiosoff, Giorgio Alessandro

(57) **Abstract**

Method and system for the management of post-acquisition processing procedures related to biomedical images, comprising user specification of the type(s) of post-acquisition processing procedures, the processing parameters, and the post-acquisition processing of the images based on the user specifications, processing which is started and executed automatically and in background.

## Description

### Field of the Invention

The present invention is in the field of information technology for healthcare. In particular, it concerns an approach to automatically identify, among the images sent by one or more acquisition modalities to an archive, the set of images and image pairs that are eligible for a pre-defined post-acquisition process of the same images, and automatically start the post-acquisition process on the identified images and/or pairs of images in background.

The present invention covers a plurality of post-acquisition processes, among which image registration represents one of the most relevant.

Other post-acquisition processing procedures may comprise for instance processes of automated segmentation of specific categories of biological tissues, automatic calculation of quantitative maps derived from images acquired by specific protocols, or automated diagnosis processes commonly known as CAD (Computer Aided Diagnosis), and in particular detection of anomalies, such as lung nodules.

### Description of the Prior Art

Image registration is the process of transforming different sets of images into one common coordinate system, and is at the basis of many visualization and analysis techniques widely used in the context of biomedical imaging. Due to the size of the data, in biomedical imaging, registration is typically time consuming. Most biomedical image processing systems that include registration among their features, typically allow performing one or more registration processes in background, but the user has to explicitly launch, and possibly also initialize, the registration process, which is then performed in background without further need of user interaction. Although performing a registration process in the background allows the user to perform other tasks in parallel, any subsequent image analysis task depending on the registration results must wait for the background process to be completed. Therefore the generally adopted flow does not allow the physician to have the registered images readily available when she really needs them, but only after having requested them and having waited for the background process to complete. This results in a reduced efficiency of the post-acquisition processing workflow and of the overall diagnostic reporting workflow. Moreover, the computing time corresponding to the time interval between image acquisition and analysis is not exploited. This time interval is typically sufficiently large to complete the registration process before starting the analysis. These exact same issues affect other types of post-acquisition processes.

The patent US2013/0195321 A1 discloses a method for the automatic registration of pairs of images acquired from MR. The method is limited only to one processing procedure, the registration, and only one image modality, only MR. Moreover in US2013/0195321, no reference is made to the usual presence, in a radiology department, of a PACS, on the contrary the system comprises a dedicated memory where all the images are saved, consequently with economic inefficiencies. Furthermore, the architecture proposed in the aforementioned patent does not allow for the processing procedure to be carried out on images deriving from different modalities, therefore excluding the possibility of any multi-modal processing, such as for instance the fusion of MR images with CT-PET images.

### Summary of the invention

In view of the disadvantages of manually launching a registration process at the beginning of an analysis task that requires such a process, the object of the present invention is to automatically initiate the registration process without waiting for the user input. In such a way, when a user starts an image analysis task, the results of the registration of the images or image sets, which the user intends to analyze, are already available in the same archive (from now on referred as PACS: Picture Archiving and Communication System) where the images are stored. Such a method determines as a result an overall reduction of the time required for the analysis task to be later completed by the user.

The present invention concerns a computer-implemented method for automatically performing the following activities:
1) Identifying, among the biomedical images stored or being stored in an archive and deriving from one or more acquisition modalities, those images or image pairs which are eligible for registration or any other post-acquisition processing, where eligible means compatible with a type/types of image analysis that requires registration. It is essential for this process to take place without loading the PACS with any additional significant activity with respect to the usual operating conditions.
2) sending the corresponding registration commands to a processing server in charge of the registration process.
3) store on the PACS the results of the performed registration processes.

The registration process can thus be applied either to single images, as for multi-parametric and/or dynamic images, such as 4d images, or pairs of images.

The proposed method is intended to work for any type of biomedical images possibly including but not limited to DICOM images from one or more of but not restricted to the following image acquisition devices: a positron emission tomography (PET) device; an x-ray computed tomography (CT) device; a magnetic resonance imaging (MRI) device; an ultrasound (US) device and other devices capable of acquiring biomedical images either in DICOM format or other formats. If the post-acquisition processing procedure is registration, the proposed method includes archiving the results and metadata of the registration process either as DICOM Spatial Registration Storage SOP or DICOM Deformable Spatial Registration Storage SOP depending on the type of transformation used in the registration process, further depending on the parameters defined by the users.

The proposed system can use standard DICOM transactions for all communication with the image acquisition devices, the PACS and the processing server.

Currently, the methods that require manual starting of the post-acquisition process require that the user manually inputs the processing commands that identify and define:
a) The image or image pairs
b) The processing parameters to be executed (for instance, in the case of registration, the parameters can be the type of transformation and the optimization options).

Conversely, the proposed method automatically extracts the set of registration commands compatible with all images contained in the archive or being archived. This operation is performed in background without any intervention by the user, made exception for the initial system configuration: in such configuration phase the user specifies the type/types of post-acquisition processes that shall be performed on the images contained and/or transmitted to the PACS.

Such a specification can be explicit or implicit. In the explicit specification the user explicitly indicates the type or types of post-acquisition processes to be performed and the processing parameters, whereas in the implicit specification the user indicates the type or types of analysis that might be performed on the images after the post-acquisition process. From the implicit specification of the analysis type/types, the method automatically identifies the type or types of post-acquisition processes required in order to later perform the analysis.

In an embodiment, the system can learn how to perform a selection of images to be processed. To this aim, the system can be trained by a user to learn the criteria to be applied in order to perform the selection. The training session can use exemplary image couples indicated by the user, images already stored and processed, and means like neural networks, genetic algorithms, bayesan networks, clustering or other machine learning algorithms known in the art.

The analysis types that can be performed - and the corresponding post-acquisition processes - can be one or more but not restricted to:
a) *longitudinal comparison* (analysis of images of the same patient acquired with the same modality and acquisition protocol, though at different times; in this case the required post-acquisition process is a registration process between two images);
b) *motion compensation* (analysis required for image series obtained through dynamic acquisition - such as Dynamic contrast enhanced MRI - requiring motion compensation through registration; in this case the required post acquisition process is a single image registration);
c) *Fusion* (analysis often applied to multi-parametric - same modality but different acquisition protocol - or a multi-modal study of the same patient; in this case the required post acquisition process is a registration process between two images);.

The specification, explicit or implicit, of the desired post-acquisition processes during configuration of the proposed system allows to perform only those post-acquisition processes strictly necessary for the analysis tasks typically performed in the healthcare facility or research lab where the system is installed.

Based on the user specification, the proposed method automatically extracts a list of comparison rules and associated processing parameters, and constantly searches, among the images stored or being stored in the PACS, those images or image pairs matching the comparison rules.

A similar procedure is disclosed in the patent US2013/0195321 A1 mentioned above, although with limits in the type of processing procedure, which is limited to the registration, and to the image modality, only MR. In such patent moreover no reference is made to the presence of a PACS, but the disclosed system comprises a dedicated memory where all the images are saved. The need of having a dedicated memory may lead to great economic inefficiencies, since the amount of data stored in a PACS typically is very high and requires heavy investments in infrastructures, if it is a PACS housed and managed by the medical unit, or in services, in the case of a Cloud PACS with outsourced management. Therefore it is preferred to use the PACS already used by the unit without replicating the images in a local memory.

However this need, arising from economic reasons, has functional drawbacks: 1) the interrogation of a PACS, that typically resides in dedicated machines if not remote ones, generates a slowdown in the execution of the processing procedure compared with the possibility of locally accessing the data 2) the large amount of interrogations performed on the PACS can easily lead to overloading the PACS that therefore gets slowed down and inefficient in satisfying the requests made by other users connected to the PACs.

Therefore in order to overcome the economic drawbacks related to the use of a memory that partially replicates the PACS and, at the same time in order to avoid functional drawbacks related to repeated interrogations to the PACS, we propose a further specification of the proposed method, comprising the interposition of a Work Flow Manager (WFM) between the PACS and the image acquisition devices. The main characteristic of the WFM is that information necessary to determine images eligible for the processing are not obtained by means of requests made to PACS but they are already present in the WFM, that keeps a local copy only of the metadata of DICOM files present in the PACS, overcoming the functional and economic drawbacks described above. By means of the WFM the interaction with PACS is limited to the recovery only of the images actually determined by the WFM as eligible in order to process them.

These and other characteristics of the present invention will be clarified from the following description of some working examples illustrated in the attached figures, in which:
Figure 1 illustrates a scheme of a preferred working example of the method that is object of the present invention.
Figure 2 illustrates a scheme of the system that is object of the present invention.

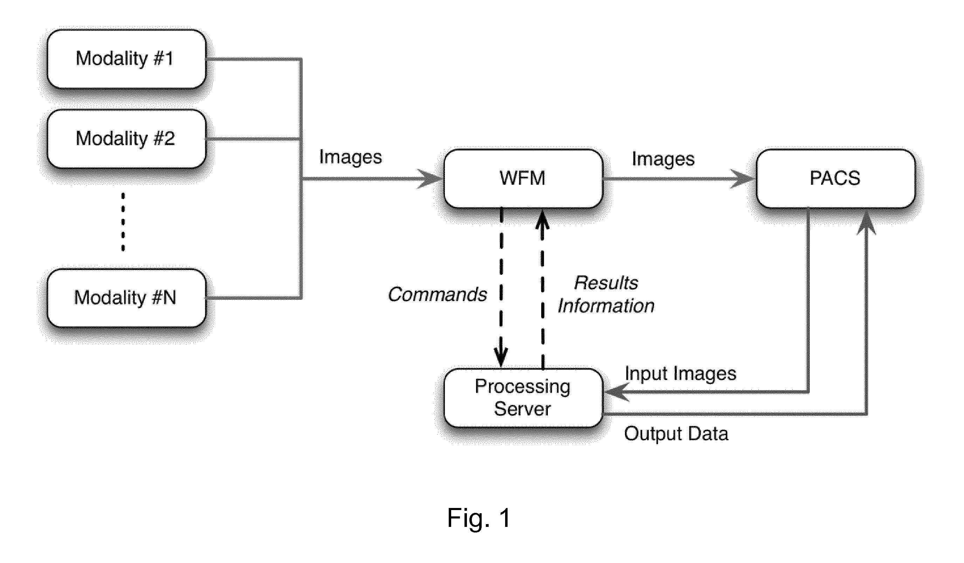

Figure 1 illustrates the use of a Work Flow Manager (WFM) between the PACS and the image acquisition devices, in which the WFM acts in the following way:
   1) the image acquisition devices send all newly acquired biomedical images to the WFM rather than to the PACS using the same communication protocol; in such a way the interposition of the WFM is completely indifferent for the image acquisition devices.
   2) The WFM reads and internally stores the image metadata for further processing and stores the images on the PACS.
   3) For each newly acquired image series, the WFM performs the following activities:
      a. Create an empty list of processing commands
      b. Checks if the image is eligible for the post-acquisition process for single images according to the matching rules (such matching rules are obtained from the user configuration); eventually associates to the image a set of processing parameters (also such processing parameters are obtained from the user configuration); add the corresponding processing command to the list;
      c. Based on the matching rules (such matching rules are obtained from the user configuration), checks if, among the archived images, there is any image eligible for the post-acquisition processing with newly archived image; such a search is performed by simply reading the image metadata which are stored in the WFM, thus without involving the PACS;
      d. associates each image pair with a set of processing parameters automatically obtained from the user configuration, and consequently updates the list of processing commands;
      e. sends the list of processing commands to the processing server in charge of the post-acquisition process;
   4) the WFM receives from the processing server the notifications on the processing progress, and the metadata of the results of the post-acquisition process which have been completed. Such metadata are stored into the WFM, whereas the process results are stored directly into the PACS by the processing server.
   5) The WFM stores the information relative to the completed registration processes as the metadata of the registration results.
   6) The WFM keeps track of the processing requests that still need to be served

The method is computer-implemented and runs wholly automatically, i.e. without any manual interaction by a user.

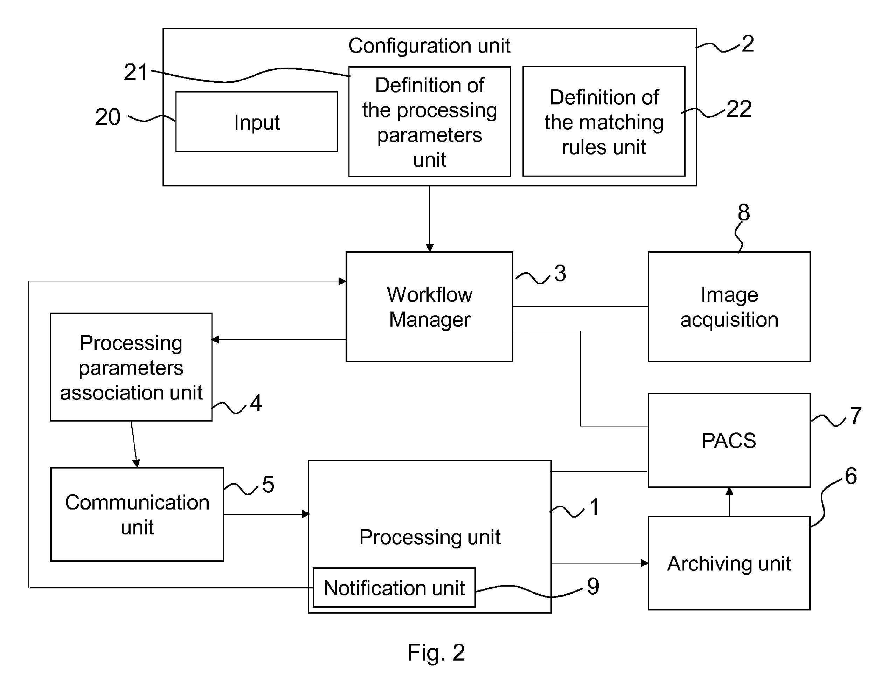

Figure 2 illustrates a working examples of the system for the automatic starting and execution of post-acquisition processes of biomedical images in background according to the present invention comprising: a post-acquisition processing unit 1 and a configuration unit 2, aimed at receiving from the user the specification of the type of post-acquisition processes and of one or more corresponding processing parameters.

The system is configured in such a way that the starting and execution of the post-acquisition processes are automatic and in background.

The processing unit 1 can be local or preferably on a remote server.

The system comprises a Work Flow Manager (WFM) 3 for the continuous identification, among the images already stored in the image archiving system PACS 7, of the images or image pairs that, based on the user configuration, are eligible for the post-acquisition process;
- A unit 4 of association of each identified image or image pair with a set of processing parameters, based on the user configuration;
- A unit 5 communicating to the processing unit 1 the processing commands relative to each identified image or image pair and corresponding processing parameters;
- An archiving unit 6, archiving the results of the post-acquisition process into the image archiving system PACS 7.

The processing unit 1 is capable of performing in parallel and at the same time several post-acquisition processes.

The biomedical images comprise but are not limited to images in DICOM format acquired from one or more of but not restricted to the following image acquisition devices: a positron emission tomography (PET) device; an x-ray computed tomography (CT) device; a magnetic resonance imaging (MRI) device; an ultrasound (US) device and other devices capable of acquiring biomedical images either in DICOM format or other formats.

The configuration unit 2 comprising:
- An input unit 20 receiving from the user the type or types of post-acquisition processing procedures to be performed on the images.
- A unit 22 of definition of the matching rules necessary for identifying the images or image pairs, based on the types of post-acquisition processing procedures received in input by the user.
- A unit 21 of definition of the processing parameters based on the types of post-acquisition processing procedures received in input by the user.

The input of the type of post-acquisition processing procedure to be executed can be either in a direct or indirect way. In the direct input the user explicitly inputs the type or types of post-acquisition processes to be performed, whereas in the indirect input the user inputs the type or types of analysis that he/her might perform on the images after the post-acquisition process. From the indirect input the Configuration Unit 2 automatically identifies the type or types of post-acquisition processes to be executed as required in order to later perform the analysis.

The analysis types accepted by the Input Unit 21 can be one or more but not restricted to: longitudinal comparison, motion compensation, fusion.

The system that automatically elaborates the type of analysis received in input by the user and extracts a list of matching rules for the identification of images or image pairs, comprising but not limited to:
1. For Longitudinal Comparison. Two images are eligible for the post-acquisition processing procedure known as registration if the PatientID, Modality and ProtocolName DICOM tags are the same.
2. Motion Compensation. A single image series is eligible for the post-acquisition processing procedure known as registration if the Modality and ProtocolName DICOM tags match those identifying a type of dynamic acquisition (such as DCE-MRI) requiring motion compensation through registration.
3. Fusion. An images series is eligible for the post-acquisition processing procedure known as registration if the StudyDescription DICOM tag matches those identifying a multi-parametric or a multi-modal study requiring registration for later fusion of the different series.

In a preferred working example, the post-acquisition processing procedure comprises the registration of the identified images. In a preferred working examples where the post-acquisition process in registration, the processing parameters comprise the type of transformation and the optimization options.

If the post-acquisition processing procedure is registration, the results and metadata of the registration process may be archived in the PACS 7 either as DICOM Spatial Registration Storage SOP or DICOM Deformable Spatial Registration Storage SOP, the choice of the archiving format depends on the configuration of the type of transformation used in the registration process and reflects the processing parameters that are part of the processing command.

The Work Flow Manager 3 which
1) receives in input a set of biomedical images directly from the image acquisition devices 8
2) reads and internally stores the image metadata for further processing and stores the images on the PACS 7.
3) For each newly acquired image:
   a. Identifies, through the comparison of the metadata stored in the WFM, the images eligible for the post-acquisition process, compatibly with the aforementioned matching rules;
   b. associates each image and/or image pair with a set of processing parameters.

Thus for each newly acquired image, the WFM checks if the image as a single image is eligible for the post-acquisition process and if other images previously archived in the PACS 7 are eligible for the post-acquisition process with the new image. In such a way a list is extracted of image and/or image pairs that must undergo the post-acquisition process.
4) The WFM 3 stores the information relative to the completed registration processes as the metadata of the registration results.
5) The WFM 3 sends the list of image identifiers and the corresponding processing parameters to the processing Unit 1;
6) the WFM receives from the processing unit 1 and internally stores the metadata of the results of the post-acquisition process and send to the processing unit 1 the command of archiving the entire process result into the PACS 7.

The processing unit 1 comprises a notification unit 9 that notifies the process progression to the WFM 3.

The processing unit 1 can accept from the WFM 3 additional processing requests, which can be executed by the processing unit 1 either in parallel with or after the completion of the ongoing process, based on the configuration of the processing unit 1.

## Claims

1. A method for the management of post-acquisition processes relative to biomedical images comprising:
a) user specification either explicit or implicit of the type or types of post-acquisition processes and processing parameters;
b) definition of a list of matching rules for identifying images or image pairs based on the type or types of post-acquisition processes received in input by the user;
c) post-acquisition processing of the images based on user specification
**characterized in that**
the starting and execution of the post-acquisition processes are automatic and in background.

2. Method, according to claim 1, in which the implicit specification comprises an indication from the user of the type or types of analysis that might be performed on the images after the post-acquisition process and the method automatically identifies the type or types of post-acquisition processes required in order to later perform the analysis.

3. Method, according to claim 1 or 2, in which the images or image pairs eligible for specified type or types of post-acquisition processes, are identified among the images stored or being stored in a picture archiving system PACS, the processes are executed and the results of the completed processes are archived.

4. Method, according to one or more of the preceding claims, comprising the following steps:
a) Identification among the images stored or being stored in a picture archiving system PACS of the images or image pairs eligible for the type or types of post-acquisition processes, based on the user specification;
b) Association to each identified image or image pair of the processing parameters based on the user specification;
c) Communication to a processing unit of the processing commands relative to each identified image or image pair and corresponding processing parameters;
d) Archiving of the post-acquisition process results into the picture archiving system PACS.

5. Method, according to one or more of the previous claims, in which the implicit user specification of the type or types of post-acquisition processes comprises the following steps:
a) Selection by the user of the type or types of analysis that the user might perform on the images following the post-acquisition process;
b) Definition of the type of post-acquisition process and of a list of processing parameters based on the user input.

6. Method, according to one or more of the previous claims, in which the identification of the images or image pairs eligible for the specified post-acquisition process is performed by a Work Flow Manager (WFM), which receives in input the biomedical images directly from the image acquisition devices, reads and internally stores the metadata of the images, stores the complete images in the picture archiving system PACS and for each newly acquired image:
a) checks if the image itself is eligible for the specified post-acquisition processes which apply to single images according to the aforementioned matching rules, and identifies, among the archived images, those that, together with the newly acquired image are eligible for the specified post-acquisition process which apply to image pairs according to the aforementioned matching rules
b) associates each identified image or image pair with a set of processing parameters;
c) sends the list of image identifiers and the corresponding processing commands to the post-acquisition processing unit;

7. Method, according to one or more of the previous claims, in which the WFM receives from the processing unit the metadata of the results of the post-acquisition process and sends to the processing unit the command to archive the entire process results into the picture archiving system PACS.

8. Method, according to one or more of the previous claims, in which the processing unit may accept additional requests of performing further post-acquisition processes from the Work Flow Manager (WFM) unit. Such additional requests are performed either in parallel or following the completion of the ongoing post-acquisition process, based on the configuration of the processing unit.

9. A system for the management of post-acquisition processes relative to biomedical images comprising a post-acquisition processing unit which can be configured through the specification of the type or types of post-acquisition processes and eventually of one or more processing parameters,
**characterized in that**
it is configured in a such way that the starting and execution of the post-acquisition processes is automatic and in background.

10. System, according to claim 9, comprising a picture archiving system PACS, in which the images or image pairs eligible for specified type or types of post-acquisition processes are identified among the images stored or being stored in the PACS, the processes being executed and the results of the completed processes being archived in the PACS.

11. System, according to claim 9 or 10, comprising
a) A Work Flow Manager (WFM) unit for the continuous identification, among the images archived in the picture archiving system PACS, the images or image pairs that are eligible for the post-acquisition processes based on the user configuration;
b) An association unit associating each identified image or image pair with a set of processing parameters, based on the user configuration;
c) A communication unit communicating to the processing unit the processing commands relative to each identified image or image pair and their corresponding processing parameters;
d) An archiving unit which archives the results of the post-acquisition process into the picture archiving system PACS.

12. System, according to one or more of claims 9 to 11, **characterized by** the fact that it acts according to the method comprising the characteristics according to one or more of claims 1 to 8.
